# EUROPEAN PATENT SPECIFICATION

(11) **EP 3 147 274 B1**
(45) Date of publication and mention of the grant of the patent: **05.09.2018**
(21) Application number: 16194071.3
(22) Date of filing: 19.02.2009
(51) Int. Cl.: C07C 17/386, C07C 21/18, C01B 7/19, C07C 17/38, C07C 17/383

(54) **PROCESSES FOR SEPARATION OF 3,3,3-TRIFLUOROPROPENE AND HYDROGEN FLUORIDE**
VERFAHREN ZUR TRENNUNG VON 3,3,3-TRIFLUORPROPEN UND FLUORWASSERSTOFF
PROCÉDÉS DE SÉPARATION DE TRIFLUOROPROPÈNE 3,3,3 ET D'ACIDE FLUORHYDRIQUE

(30) Priority: 21.02.2008 US 30365 P
(43) Date of publication of application: 29.03.2017
(62) Divisional of application: 09711602.4
(73) Proprietor: The Chemours Company FC, LLC, Wilmington DE 19801 (US)
(72) Inventor: KNAPP, Jeffrey, P., Wilmington, DE 19808 (US)
(74) Representative: Dehns

(56) References cited:
- WO-A1-2008/024508
- US-A1- 2006 106 263
- US-A1- 2007 100 175

## Description

### BACKGROUND INFORMATION

### Field of the Disclosure

This disclosure relates in general to processes for separating HF from fluoroolefins.

### Description of the Related Art

The chemical manufacture of fluoroolefins may produce mixtures of the desired fluoroolefins and hydrogen fluoride (HF). The separation of fluoroolefins and HF is not always easily accomplished. Existing methods of distillation and decantation are very often ineffective for separation of these compounds. Aqueous scrubbing may be effective, but requires the use of large amounts of scrubbing solutions and produces excessive waste as well as wet product that must then be dried. Therefore, there is a need for new methods of separating HF from fluoroolefins.

US 2006/0106263 A1 discloses processes for producing hydrofluoroolefins, and processes for separating hydrofluoroolefins from hydrofluorocarbons and hydrogen fluoride by azeotropic distillation. One hydrofluoroolefin referred to therein is HFC-1243zf (3,3,3-trifluoropropene), but no specific azeotropes are disclosed.

### SUMMARY

The present disclosure provides a process for separating HF from a mixture comprising HFC-1243zf, HF, and at least one of HFC-254fb or HFC-254eb, said process comprising: a) adding an entrainer to the mixture comprising HFC-1243zf, HF, and at least one of HFC-254fb or HFC-254eb thus forming a second mixture; b) distilling said second mixture in a first distillation step to form a first distillate composition comprising HF and entrainer and a first bottoms composition comprising HFC-1243zf and at least one of HFC-254fb or HFC-254eb; c) condensing said first distillate composition to form two liquid phases, being (i) an entrainer-rich phase and (ii) an HF-rich phase; and d) recycling the entrainer-rich phase back to the first distillation step.

The present disclosure also provides the use of an entrainer for separating HF from a mixture comprising HFC-1243zf, HF, and at least one of HFC-254fb or HFC-254eb.

The foregoing general description and the following detailed description are exemplary and explanatory only and are not restrictive of the invention, as defined in the appended claims. The azeotrope-based processes described below are claimed in the parent applcation and are not part of the invention.

### BRIEF DESCRIPTION OF THE DRAWINGS

An embodiment is illustrated in the accompanying figure to improve understanding of concepts as presented herein.

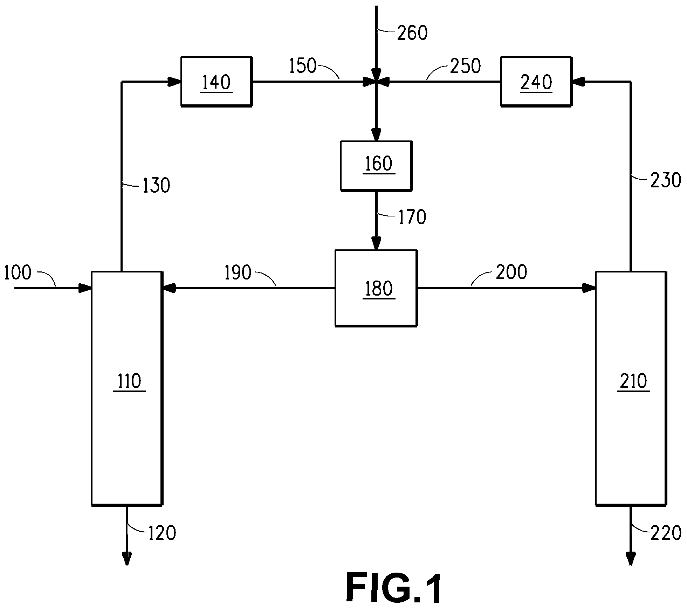

FIG. 1 is an illustration of one embodiment of a process to separate HFC-1243zf and at least one of HFC-254eb and HFC-254fb from a mixture comprising HFC-1243zf, HF and said at least one of HFC-254eb and HFC-254fb via azeotropic distillation wherein a supplemental entrainer is fed to the distillation.

Skilled artisans appreciate that objects in the figures are illustrated for simplicity and clarity and have not necessarily been drawn to scale. For example, the dimensions of some of the objects in the figures may be exaggerated relative to other objects to help to improve understanding of embodiments.

### DETAILED DESCRIPTION

Many aspects and embodiments have been described above and are merely exemplary and not limiting. After reading this specification, skilled artisans appreciate that other aspects and embodiments are possible without departing from the scope of the invention.

Other features and benefits of any one or more of the embodiments will be apparent from the following detailed description, and from the claims.

### 1. Definitions and Clarification of Terms

Before addressing details of embodiments described below, some terms are defined or clarified.

Hydrogen fluoride (HF, anhydrous) is a commercially available chemical or can be produced by methods known in the art.

3,3,3-trifluoropropene (HFC-1243zf, CF₃CH=CH₂) may be prepared by known methods, such as dehydrofluorination of 1,1,1,2-tetrafluoropropane (CF₃CH₂CH₂F or HFC-254fb) or 1,1,1,3-tetrafluoropropane (CF₃CHFCH₃ or HFC-254eb). HFC-254fb is available commercially or may be made by the reaction of CIF with CCl₃CH₂CH₂Cl (N. N. Chuvatkin, et al, Zh. Org. Khim., 18 (1982) 946), by the fluorination of CF₃CH₂CH₂I with HgF, and via Zn reduction of CF₃CH₂CHFI (R. N. Hazeldine, et al, J. Chem. Soc., (1953) 1199) or CF₃CH₂CHFBr (P. Tarrant, et al, J. Am. Chem. Soc., 77 (1955) 2783). HFC-254eb is available commercially or may be made by the addition of fluoromethane and trifluoroethylene with an antimony pentafluoride catalyst, as described in for instance, US 6,184,426.

HFC-1243zf may also be made by fluorination of 1,1,1,3-tetrachloropropane (CCl₃CH₂CH₂Cl or HCC-250fb) with hydrogen fluoride over a fluorination catalyst such as chromium/alumina fluoride or chromium oxide catalysts. HCC-250fb may be made by processes known in the art such as described in US 4,605,802 and US 5,705,779 by an addition reaction of carbon tetrachloride and ethylene.

HFC-1243zf has been found to form a binary azeotropic composition with HF. The azeotropic composition comprises about 72.0 mole % HFC-1243zf and about 28.0 mole % HF at 29.8 °C and 106.6 psia (735 kPa). Further, the azeotropic composition comprises about 76.2 mole % HFC1243zf and about 23.8 mole % HF at 79.7 °C and 363 psia (2503 kPa).

The process equipment for all the processes disclosed herein and the associated feed lines, effluent lines and associated units may be constructed of materials resistant to hydrogen fluoride. Typical materials of construction, well-known to the art, include stainless steels, in particular of the austenitic type, and the well-known high nickel alloys such as Monel® nickel-copper alloys, Hastelloy® nickel based alloys and Inconel® nickel-chromium alloys.

Azeotropic distillation is a process in which a distillation column is operated under conditions to cause one or more azeotropic or azeotrope-like composition to form, and thereby facilitates the separation of the components of the mixture. Azeotropic distillations may occur where only the components of the mixture to be separated are distilled, or where an entrainer is added that forms an azeotrope with one or more of the components of the initial mixture. Entrainers that act in this manner, that is to say, that form an azeotrope with one of more of the components of the mixture to be separated thus facilitating the separation of those components by distillation, are more commonly called azeotroping agents or azeotropic entrainers.

In conventional or azeotropic distillations, the overhead or distillate stream exiting the column may be condensed using conventional reflux condensers. At least a portion of this condensed stream can be returned to the top of the column as reflux, and the remainder recovered as product or for optional processing. The ratio of the condensed material which is returned to the top of the column as reflux to the material removed as distillate is commonly referred to as the reflux ratio. The compounds and entrainer exiting the column as distillate or distillation bottoms stream can then be passed to a stripper or second distillation column for separation by using conventional distillation, or may be separated by other methods, such as decantation. If desired, the entrainer may then be recycled back to the first distillation column for reuse.

The specific conditions which can be used for practicing the invention depend upon a number of parameters, such as the diameter of the distillation column, feed points, number of separation stages in the column, among others. In one embodiment, the operating pressure of the distillation system may range from about 5 to 500 psia (0.5 to 50.5 MPa); in another embodiment, about 20 to 400 psia (2 to 40.5 MPa). Normally, increasing the reflux ratio results in increased distillate stream purity, but generally the reflux ratio ranges between 1/1 to 200/1. The temperature of the condenser, which is located adjacent to the top of the column, is normally sufficient to substantially fully condense the distillate that is exiting from the top of the column, or is that temperature required to achieve the desired reflux ratio by partial condensation.

The problems associated with conventional distillation can be solved by a distillation process using entrainers. The difficulty in applying this method is that there is no known way, short of experimentation, of predicting which if any compound will be an effective entrainer.

As used herein, by "essentially free of" is meant that a composition contains less than about 100 ppm (mole basis), less than about 10 ppm or less than about 1 ppm, of the specified component. If a composition is essentially free of more than one component, then the total concentration of those components is less than about 100 ppm, less than about 10 ppm, or less than about 1 ppm.

Hydrogen fluoride (HF, anhydrous) is a commercially available chemical or can be produced by methods known in the art.

By entrainer is meant any compound that, when added to a first mixture, forms one or more azeotropes with the components of the mixture to facilitate separation of the components of the mixture. As used herein, the terms "entrainer" and "entraining agent" are used interchangeably and are to be interpreted as having identical meaning.

The term "entrainer" is used herein to describe any compound that would be effective in separation of fluoroolefins from mixtures comprising HF and fluoroolefin in an azeotropic distillation process. Included as useful entrainers are those compounds that form azeotropes with one or more of the components of a mixture, including fluoroolefins, HF, and possible hydrofluorocarbons for which the boiling point of at least one of such azeotropes is lower than the boiling point of the fluoroolefin/HF azeotrope.

Entrainers may be selected from the group consisting of hydrocarbons, chlorocarbons, chlorofluorocarbons, hydrochlorofluorocarbons, hydrofluorocarbons, perfluorocarbons, fluoroethers, HFPO, SF₆, chlorine, hexafluoroacetone, and mixtures thereof.

Hydrocarbon entrainers comprise compounds containing 1 to 5 carbon atoms and hydrogen. Hydrocarbon entrainers may be linear, branched, cyclic, saturated or unsaturated compounds. Representative hydrocarbon entrainers include but are not limited to methane, ethane, ethylene, acetylene, vinylacetylene, propane, propylene, propyne, cyclopropane, cyclopropene, propadiene, n-butane, isobutane, 1-butene, isobutene, 1,3-butadiene, 2,2-dimethylpropane, cis-2-butene, trans-2-butene, 1-butyne, n-pentane, isopentane, neopentane, cyclopentane, 1-pentene, 2-pentene, and mixtures thereof.

Chlorocarbon entrainers comprise compounds containing carbon, chlorine and optionally hydrogen, including but not limited to methylene chloride (CH₂Cl₂), and methyl chloride (CH₃Cl).

Chlorofluorocarbon (CFC) entrainers comprise compounds with carbon, chlorine and fluorine. Representative CFCs include but are not limited to dichlorodifluoromethane (CFC-12), 2-chloro-1,1,2-trifluoroethylene, chloropentafluoroethane (CFC-115), 1,2-dichloro-1,1,2,2-tetrafluoroethane (CFC-114), 1,1-dichloro-1,2,2,2-tetrafluoroethane (CFC-114a), 1,1,2-trichloro-1,2,2-trifluoroethane (CFC-113), 1,1,1-trichloro-2,2,2-trifluoroethane (CFC-113a), 1,1,2-trichloro-1,2,3,3,3-pentafluoropropane (CFC-215bb), 2,2-dichloro-1,1,1,3,3,3-hexafluoropropane (CFC-216aa), 1,2-dichloro-1,1,2,3,3,3-hexafluoropropane (CFC-216ba), 2-chloro-1,1,1,2,3,3,3-heptafluoropropane (CFC-217ba), 2-chloro-1,1,3,3,3-pentafluoropropene (CFC-1215xc), and mixtures thereof.

Hydrochlorofluorocarbon (HCFC) entrainers comprise compounds with carbon, chlorine, fluorine and hydrogen. Representative HCFCs include but are not limited to dichlorofluoromethane (HCFC-21), 1,1-dichloro-3,3,3-trifluoroethane (HCFC-123), 1,1-dichloro-1-fluoroethane (HCFC-141b), 2-chloro-1,1,1,2-tetrafluoroethane (HCFC-124), 1-chloro-1,1,2,2-tetrafluoroethane (HCFC-124a), 2-chloro-1,1,1-trifluoroethane (HCFC-133a), 1-chloro-1,1-difluoroethane (HCFC-142b), 2-chloro-1,1-difluoroethylene (HCFC-1122), and mixtures thereof.

Hydrofluorocarbon (HFC) entrainers comprise compounds that contain carbon, hydrogen and fluorine. Representative HFCs include but are not limited to 1,1,2-trifluoroethylene (HFC-1123), 1,1-difluoroethylene (HFC-1132a), 2,3,3,3-tetrafluoropropene (HFC-1234yf), and mixtures thereof.

Perfluorocarbon (PFC) entrainers comprise compounds with carbon and fluorine only. Representative PFCs include but are not limited to hexafluoroethane (PFC-116), octafluoropropane (PFC-218), 1,1,1,4,4,4-hexafluoro-2-butyne (PFBY-2), hexafluoropropylene (HFP, PFC-1216), hexafluorocyclopropane (PFC-C216), octafluorocyclobutane (PFC-C318), decafluorobutane (PFC-31-10, any isomer(s)), 2,3-dichloro-1,1,1,4,4,4-hexafluoro-2-butene (PFC-1316mxx), octafluoro-2-butene (PFC-1318my, cis and trans), hexafluorobutadiene (PFC-2316), and mixtures thereof.

Fluoroether entrainers comprise compounds with carbon, fluorine, optionally hydrogen and at least one ether group oxygen. Representative fluoroethers include but are not limited to trifluoromethyl-difluoromethyl ether (CF₃OCHF₂, HFOC-125E), 1,1-difluorodimethyl ether, tetrafluorodimethylether (HFOC-134E), difluoromethyl methyl ether (CHF₂OCH₃, HFOC-152aE), pentafluoroethyl methyl ether, and mixtures thereof.

Miscellaneous other compounds that may be useful as entrainers include HFPO (hexafluoropropylene oxide), chlorine (Cl₂), hexafluoroacetone, PMVE (perfluoromethylvinylether), PEVE (perfluoroethyl vinyl ether), and mixtures thereof.

Entrainers as described above are available commercially or may be produced by methods known in the art.

As used herein, the terms "comprises," "comprising," "includes," "including," "has," "having" or any other variation thereof, are intended to cover a non-exclusive inclusion. For example, a process, method, article, or apparatus that comprises a list of elements is not necessarily limited to only those elements but may include other elements not expressly listed or inherent to such process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

Also, use of "a" or "an" are employed to describe elements and components described herein. This is done merely for convenience and to give a general sense of the scope of the invention. This description should be read to include one or at least one and the singular also includes the plural unless it is obvious that it is meant otherwise.

Unless otherwise defined, all technical and scientific terms used herein have the same meaning as commonly understood by one of ordinary skill in the art to which this invention belongs. Although methods and materials similar or equivalent to those described herein can be used in the practice or testing of embodiments of the present invention, suitable methods and materials are described below. In case of conflict, the present specification, including definitions, will control. In addition, the materials, methods, and examples are illustrative only and not intended to be limiting.

### 2. Separation of HFC-254 from HFC-1243zf and HF

As noted above, HFC-1243zf may be produced by dehydrofluorination of certain HFC-254 (tetrafluoropropane) isomers. By HFC-254 is meant any isomer of tetrafluoropropane and any combinations of any isomers of tetrafluoropropane that can yield HFC-1243zf upon dehydrofluorination. Isomers of tetrafluoropropane include HFC-254eb (1,1,1,2-tetrafluoropropane) and HFC-254fb (1,1,1,3-tetrafluoropropane).

An entrainer may be added to enable separation of the resulting HF from the HFC-1243zf and HFC-254eb and/or HFC-254fb.

For example, the mixture of HF, HFC-1243zf, HFC-254eb and/or HFC-254fb may be formed by any practical means, such as by feeding at least one of HFC-254fb or HFC-254eb over a chrome oxide catalyst at elevated temperature. The mixture of HF, HFC-1243zf, HFC-254eb and/or HFC-254fb may be fed to a distillation column. A suitable entrainer is then also fed to the distillation column, either as a separate stream or by being mixed in with the HF/HFC-1243zf/HFC-254fb and/or HFC-254eb mixture prior to feeding it to the distillation column. The distillation column is then operated under conditions sufficient to form a low-boiling azeotrope composition between the entrainer and HF, with the HF and entrainer removed as the column distillate, and the HFC-1243zf, HFC-254eb and/or HFC-254fb recovered from the column bottoms essentially free of HF. The HFC-1243zf may then be separated from the HFC-254eb and/or HFC-254fb by any usual means including conventional distillation, with the HFC-1243zf being recovered as product and with the HFC-254eb and/or HFC-254fb optionally being recycled back to the reaction step to produce HFC-1243zf.

Thus the invention provides a process for separating HF from a mixture comprising HFC-1243zf, HF, and at least one of HFC-254eb or HFC-254fb. The process comprises:
a. adding an entrainer to the mixture comprising HFC-1243zf, HF, and at least one of HFC-254eb or HFC-254fb thus forming a second mixture;
b. distilling said second mixture in a first distillation step to form a first distillate composition comprising HF and entrainer and a first bottoms composition comprising HFC-1243zf and at least one of HFC-254eb or HFC-254fb;
c. condensing said first distillate composition to form two liquid phases, being (i) an entrainer-rich phase and (ii) an HF-rich phase; and
d. recycling the entrainer-rich phase back to the first distillation step.

In another embodiment, the process may further comprise feeding the HF-rich phase to a second distillation step and forming a second distillate composition comprising an azeotrope of entrainer and HF and a second bottoms composition comprising HF essentially free of entrainer. In another embodiment, the process may further comprise recycling said second distillate composition back to the two liquid phases.

Referring now to FIG 1, a stream comprising HF, HFC-1243zf, and at least one of HFC-254eb or HFC-254fb is fed to a first distillation column 110 via stream 100. An entrainer-rich stream is also fed to this column via stream 190. Column 110 is operated under conditions to cause HF to distill overhead with the entrainer due to the influence of the low-boiling HF/entrainer azeotrope. Sufficient entrainer is fed to this first column via stream 190 such that HFC-1243zf and HFC-254eb or HFC-254fb may be obtained essentially free of entrainer and HF as the bottoms from column 110 via stream 120. The HFC-1243zf and HFC-254eb or HFC-254fb in stream 120 may then optionally be separated from each other by conventional distillation and the HFC-254eb or HFC-254fb optionally recycled back to a dehydrofluorination reactor to form HFC-1243zf. The distillate from column 110, removed via stream 130, contains essentially all of the entrainer and HF in column feeds 100 and 190 and, optionally, some HFC-254eb or HFC-254fb and/or HFC-1243zf. This first distillate stream 130 is condensed by condenser 140 to form stream 150, which is then mixed with condensed distillate stream 250 from the second distillation column and, as needed, additional fresh entrainer added via stream 260. This combined stream is sub-cooled by cooler 160 and sent via stream 170 to decanter 180 where it separates into separate entrainer-rich and HF-rich liquid fractions which are removed via streams 190 and 200, respectively. The majority of the HFC-254eb or HFC-254fb and HFC-1243zf present in the decanter partition into the entrainer-rich phase fraction. The entrainer-rich fraction is fed to the first distillation column 110 via stream 190. The HF-rich fraction from the decanter is fed via stream 200 to a second distillation column 210 containing 8 theoretical stages and operated under conditions such that a bottoms stream of HF essentially free of HFC-254eb or HFC-254fb, HFC-1243zf, and entrainer is produced and removed via stream 220. The distillate from column 210, removed via stream 230 and containing essentially all of the HFC-254eb or HFC-254fb, HFC-1243zf, and entrainer present in the column feed (stream 200) plus the HF not recovered in product stream 220, is condensed by condenser 240 and removed via stream 250. Condensed distillate stream 250 is combined with both the condensed distillate stream 150 from the first column and, as needed, fresh entrainer, added via stream 260, then cooled and fed to the decanter for further separation.

In one embodiment, entrainers for HF separation from HFC-1243zf and HFC-254eb and/or HFC-254fb include: methane, ethane, ethylene, acetylene, vinylacetylene, propane, propylene, propyne, cyclopropane, cyclopropene, propadiene, methyl chloride (CH₃Cl), dichlorodifluoromethane (CFC-12), 2-chloro-1,1,2-trifluoroethylene, chloropentafluoroethane (CFC-115), 2-chloro-1,1,3,3,3-pentafluoropropene (CFC-1215xc), 2-chloro-1,1-difluoroethylene (HCFC-1122), 1,1,2-trifluoroethylene (HFC-1123), 1,1-difluoroethylene (HFC-1132a), 2,3,3,3-tetrafluoropropene (HFC-1234yf), hexafluoroethane (PFC-116), octafluoropropane (PFC-218), 1,1,1,4,4,4-hexafluoro-2-butyne (PFBY-2), hexafluoropropylene (HFP, PFC-1216), hexafluorocyclopropane (PFC-C216), trifluoromethyl-difluoromethyl ether (CF₃OCHF₂, HFOC-125E), 1,1-difluorodimethyl ether, tetrafluorodimethylether (HFOC-134E), difluoromethyl methyl ether (CHF₂OCH₃, HFOC-152aE), pentafluoroethyl methyl ether, HFPO, chlorine (Cl₂), hexafluoroacetone, PMVE (perfluoromethylvinylether), PEVE (perfluoroethylvinylether), and mixtures thereof.

In another embodiment, the entrainer that is effective for separation of HF from HFC-1243zf and HFC-254eb and/or HFC-254fb comprises propane.

### EXAMPLES

The concepts described herein will be further described in the following examples, which do not limit the scope of the invention described in the claims.

### EXAMPLE 1

This Example shows one way in which HF may be separated from HFC-1243zf and HFC-254fb by azeotropic distillation using an added entrainer. The composition of the feed mixture is such as one might obtain from a dehydrofluorination reactor operated with partial conversion, i.e., it contains equimolar amounts of HF and HFC-1243zf and any unreacted HFC-254fb.

Referring now to FIG 1, a stream comprising HF, HFC-1243zf, and HFC-254fb is fed to the third stage from the top of a first distillation column 110 via stream 100. An entrainer-rich stream is also fed to this column via stream 190. In this example, propane is used as the entrainer.

Column 110 contains 19 theoretical stages and is operated under conditions to cause HF to distill overhead with the entrainer due to the existence of the low-boiling HF/propane azeotrope. Sufficient propane is fed to this first column via stream 190 such that HFC-1243zf and HFC-254fb may be obtained essentially free of propane and HF as the bottoms from column 110 via stream 120. The HFC-1243zf and HFC-254fb in stream 120 may then optionally be separated from each other by conventional distillation and the HFC-254fb optionally recycled back to a dehydrofluorination reactor to form HFC-1243zf. The distillate from column 110, removed via stream 130, contains essentially all of the propane and HF in column feeds 100 and 190 as well as some HFC-254fb and HFC-1243zf. This first distillate stream 130 is condensed by condenser 140 to form stream 150, which is then mixed with condensed distillate stream 250 from the second distillation column and, as needed, additional fresh propane added via stream 260. This combined stream is sub-cooled by cooler 160 and sent via stream 170 to decanter 180 where it separates into propane-rich and HF-rich liquid phase fractions which are removed via streams 190 and 200, respectively. The majority of the HFC-254fb and HFC-1243zf present in the decanter partition into the propane-rich phase fraction. The propane-rich phase fraction is fed to the top stage of the first distillation column 110 via stream 190 as reflux where it will undergo additional separation. The HF-rich fraction from the decanter is fed via stream 200 to the top stage of a second distillation column 210 containing 8 theoretical stages and operated under conditions such that a bottoms stream of HF essentially free of HFC-254fb, HFC-1243zf, and propane is produced and removed via stream 220. The distillate from column 210, removed via stream 230 and containing essentially all of the HFC-254fb, HFC-1243zf, and propane present in stream 200 plus the HF not recovered in product stream 220, has a composition approaching the HF/HFC-1243zf azeotrope, and is condensed by condenser 240 and removed via stream 250. Condensed distillate stream 250 is combined with both the condensed distillate stream 150 from the first column and, as needed, fresh entrainer, added via stream 260, then cooled and fed to the decanter for further separation.

The data in Table 1 were obtained by calculation using measured and calculated thermodynamic properties.

**TABLE 1**

| Component or variable | Feed | First col btm | First Dist | Propane-rich phase | HF-rich phase | Second col btm | Second Dist |
|---|---|---|---|---|---|---|---|
| Stream # | 100 | 120 | 130 | 190 | 200 | 220 | 230 |
| HF, wt% | 5.2 | <1 ppm | 3.6 | 0.5 | 60.8 | 100 | 51.9 |
| HFC-254fb, wt% | 70.1 | 73.9 | 0.4 | 0.4 | 0.2 | <1 ppm | 0.3 |
| HFC-1243zf, wt% | 24.7 | 26.1 | 62.8 | 64.7 | 37.4 | <1 ppm | 45.9 |
| propane, wt% | 0 | <1 ppm | 33.2 | 34.3 | 1.6 | <1 ppm | 1.9 |
| Temp, °C | 30.0 | 69.6 | 18.9 | -30.0 | -30.0 | 88.9 | 72.8 |
| Pres, psia | 116 | 116 | 115 | 115 | 115 | 116 | 115 |
| Pres. kPa | 800 | 800 | 793 | 793 | 793 | 800 | 793 |

## Claims

1. A process for separating HF from a mixture comprising HFC-1243zf, HF, and at least one of HFC-254fb or HFC-254eb, said process comprising:
a. adding an entrainer to the mixture comprising HFC-1243zf, HF, and at least one of HFC-254fb or HFC-254eb thus forming a second mixture;
b. distilling said second mixture in a first distillation step to form a first distillate composition comprising HF and entrainer and a first bottoms composition comprising HFC-1243zf and at least one of HFC-254fb or HFC-254eb;
c. condensing said first distillate composition to form two liquid phases, being (i) an entrainer-rich phase and (ii) an HF-rich phase; and
d. recycling the entrainer-rich phase back to the first distillation step.

2. The process of claim 1 further comprising feeding the HF-rich phase to a second distillation step and forming a second distillate composition comprising an azeotrope of entrainer and HF and a second bottoms composition comprising HF essentially free of entrainer.

3. The process of claim 1, wherein said entrainer is selected from the group consisting of:
a. hydrocarbon entrainers comprising at least one compound selected from the group consisting of: methane, ethane, ethylene, acetylene, vinylacetylene, propane, propylene, propyne, cyclopropane, cyclopropene, propadiene, n-butane, isobutane, 1-butene, isobutene, 1,3-butadiene, 2,2-dimethylpropane, cis-2-butene, trans-2-butene, 1-butyne, n-pentane, isopentane, neopentane, cyclopentane, 1-pentene, 2-pentene, and mixtures thereof;
b. chlorocarbon entrainers selected from the group consisting of methylene chloride, methyl chloride, and mixtures thereof;
c. chlorofluorocarbon (CFC) entrainers comprising at least one compound selected from the group consisting of: dichlorodifluoromethane (CFC-12), 2-chloro-1,1,2-trifluoroethylene, chloropentafluoroethane (CFC-115), 1,2-dichloro-1,1,2,2-tetrafluoroethane (CFC-114), 1,1-dichloro-1,2,2,2-tetrafluoroethane (CFC-114a), 1,1,2-trichloro-1,2,2-trifluoroethane (CFC-113), 1,1,1-trichloro-2,2,2-trifluoroethane (CFC-113a), 1,1,2-trichloro-1,2,3,3,3-pentafluoropropane (CFC-215bb), 2,2-dichloro-1,1,1,3,3,3-hexafluoropropane (CFC-216aa), 1,2-dichloro-1,1,2,3,3,3-hexafluoropropane (CFC-216ba), 2-chloro-1,1,1,2,3,3,3-heptafluoropropane (CFC-217ba), 2-chloro-1,1,3,3,3-pentafluoropropene (CFC-1215xc), and mixtures thereof;
d. hydrochlorofluorocarbon (HCFC) entrainers comprising at least one compound selected from the group consisting of: dichlorofluoromethane (HCFC-21), 1,1-dichloro-3,3,3-trifluoroethane (HCFC-123), 1,1-dichloro-1-fluoroethane (HCFC-141b), 2-chloro-1,1,1,2-tetrafluoroethane (HCFC-124), 1-chloro-1,1,2,2-tetrafluoroethane (HCFC-124a), 2-chloro-1,1,1-trifluoroethane (HCFC-133a), 1-chloro-1,1-difluoroethane (HCFC-142b), 2-chloro-1,1-difluoroethylene (HCFC-1122), and mixtures thereof;
e. hydrofluorocarbon (HFC) entrainers comprising at least one compound selected from the group consisting of: 1,1,2-trifluoroethylene (HFC-1123), 1,1-difluoroethylene (HFC-1132a), 2,3,3,3-tetrafluoropropene (HFC-1234yf), and mixtures thereof;
f. perfluorocarbon (PFC) entrainers comprising at least one compound selected from the group consisting of: hexafluoroethane (PFC-116), octafluoropropane (PFC-218), 1,1,1,4,4,4-hexafluoro-2-butyne (PFBY-2), hexafluoropropylene (HFP, PFC-1216), hexafluorocyclopropane (PFC-C216), octafluorocyclobutane (PFC-C318), decafluorobutane (PFC-31-10, all isomers), 2,3-dichloro-1,1,1,4,4,4-hexafluoro-2-butene (PFC-1316mxx), octafluoro-2-butene (PFC-1318my, cis and trans), hexafluorobutadiene (PFC-2316), and mixtures thereof;
g. fluoroether entrainers comprising at least one compound selected from the group consisting of: trifluoromethyl-difluoromethyl ether (CF₃OCHF₂, HFOC-125E), 1,1-difluorodimethyl ether, tetrafluorodimethylether (HFOC-134E), difluoromethyl methyl ether (CHF₂OCH₃, HFOC-152aE), pentafluoroethyl methyl ether, and mixtures thereof; and
h. at least one compound selected from the group consisting of HFPO (hexafluoropropylene oxide), SF₆, chlorine, hexafluoroacetone, PMVE (perfluoromethylvinylether), PEVE (perfluoroethylvinylether), and mixtures thereof.

4. Use of an entrainer for separating HF from a mixture comprising HFC-1243zf, HF, and at least one of HFC-254fb or HFC-254eb

## Patentansprüche

1. Verfahren zum Trennen von HF von einer Mischung umfassend HFC-1243zf, HF und mindestens eines von HFC-254fb oder HFC-254eb, wobei das Verfahren folgendes umfasst:
a. Zugeben eines Schleppmittels zu der Mischung umfassend HFC-1243zf, HF und mindestens eines von HFC-254fb oder HFC-254eb, wodurch eine zweite Mischung gebildet wird;
b. Destillieren der zweiten Mischung in einem ersten Destillationsschritt, um eine erste Destillatzusammensetzung zu bilden, die HF und Schleppmittel und eine erste Sumpfproduktzusammensetzung umfasst, die HFC-1243zf und mindestens eines von HFC-254fb oder HFC-254eb umfasst;
c. Kondensieren der ersten Destillatzusammensetzung, um zwei flüssige Phasen zu bilden, die (i) eine an Schleppmittel reiche Phase und (ii) eine an HF reiche Phase sind; und
d. Zurückführen der an Schleppmittel reichen Phase zurück zum ersten Destillationsschritt.

2. Verfahren nach Anspruch 1, weiterhin umfassend Zuführen der an HF reichen Phase zu einem zweiten Destillationsschritt und Bilden einer zweiten Destillatzusammensetzung umfassend einen Azeotrop von Schleppmittel und HF und eine zweite Sumpfproduktzusammensetzung umfassend HF, das im Wesentlichen frei von Schleppmittel ist.

3. Verfahren gemäß Anspruch 1, wobei das Schleppmittel aus der Gruppe ausgewählt wird bestehend aus:
a. Kohlenwasserstoff-Schleppmitteln umfassend mindestens eine Verbindung ausgewählt aus der Gruppe bestehend aus: Methan, Ethan, Ethylen, Acetylen, Vinylacetylen, Propan, Propylen, Propyn, Cyclopropan, Cyclopropen, Propadien, n-Butan, Isobutan, 1-Buten, Isobuten, 1,3-Butadien, 2,2-Dimethylpropan, cis-2-Buten, trans-2-Buten, 1-Butyn, n-Pentan, Isopentan, Neopentan, Cyclopentan, 1-Penten, 2-Penten und Mischungen davon;
b. Chlorkohlenstoff-Schleppmitteln ausgewählt aus der Gruppe bestehend aus Methylenchlorid, Methylchlorid und Mischungen davon;
c. Chlorfluorkohlenstoff- (CFC-) Schleppmitteln umfassend mindestens eine Verbindung ausgewählt aus der Gruppe bestehend aus: Dichlordifluormethan (CFC-12), 2-Chlor-1,1,2-trifluorethylen, Chlorpentafluorethan (CFC-115), 1,2-Dichlor-1,1,2,2-tetrafluorethan (CFC-114), 1,1-Dichlor-1,2,2,2-tetrafluorethan (CFC-114a), 1,1,2-Trichlor-1,2,2-trifluorethan (CFC-113), 1,1,1-Trichlor-2,2,2-trifluorethan (CFC-113a), 1,1,2-Trichlor-1,2,3,3,3-pentafluorpropan (CFC-215bb), 2,2-Dichlor-1,1,1,3,3,3-hexafluorpropan (CFC-216aa), 1,2-Dichlor-1,1,2,3,3,3-hexafluorpropan (CFC-216ba), 2-Chlor-1,1,1,2,3,3,3-heptafluorpropan (CFC-217ba), 2-Chlor-1,1,3,3,3-pentafluorpropen (CFC-1215xc) und Mischungen davon;
d. Chlorfluorkohlenwasserstoff- (HCFC-) Schleppmitteln umfassend mindestens eine Verbindung ausgewählt aus der Gruppe bestehend aus: Dichlorfluormethan (HCFC-21), 1,1-Dichlor-3,3,3-trifluorethan (HCFC-123), 1,1-Dichlor-1-fluorethan (HCFC-141b), 2-Chlor-1,1,1,2-tetrafluorethan (HCFC-124), 1-Chlor-1,1,2,2-tetrafluorethan (HCFC-124a), 2-Chlor-1,1,1-trifluorethan (HCFC-133a), 1-Chlor-1,1-difluorethan (HCFC-142b), 2-Chlor-1,1-difluorethylen (HCFC-1122) und Mischungen davon;
e. Fluorkohlenwasserstoff- (HFC) Schleppmitteln umfassend mindestens eine Verbindung ausgewählt aus der Gruppe bestehend aus: 1,1,2-Trifluorethylen (HFC-1123), 1,1-Difluorethylen (HFC-1132a), 2,3,3,3-Tetrafluorpropen (HFC-1234yf) und Mischungen davon;
f. Perfluorkohlenstoff- (PFC.) Schleppmitteln umfassend mindestens eine Verbindung ausgewählt aus der Gruppe bestehend aus: Hexafluorethan (PFC- 116), Octafluorpropan (PFC-218), 1,1,1,4,4,4-Hexafluor-2-butyn (PFBY-2), Hexafluorpropylen (HFP, PFC-1216), Hexafluorcyclopropan (PFC-C216), Octafluorcyclobutan (PFC-C318), Decafluorbutan (PFC-31-10, alle Isomere), 2,3-Dichlor-1,1,1,4,4,4-hexafluor-2-buten (PFC-1316mxx), Octafluor-2-buten (PFC-1318my, cis und trans), Hexafluorbutadien (PFC-2316) und Mischungen davon;
g. Fluorether-Schleppmitteln umfassend mindestens eine Verbindung ausgewählt aus der Gruppe bestehend aus: Trifluormethyldifluormethylether (CF₃OCHF₂, HFOC-125E), 1,1-Difluordimethylether, Tetrafluordimethylether (HFOC-134E), Difluormethylmethylether (CHF₂OCH₃, HFOC-152aE), Pentafluorethylmethylether und Mischungen davon; und
h. mindestens einer Verbindung ausgewählt aus der Gruppe bestehend aus HFPO (Hexafluorpropylenoxid), SF₆, Chlor, Hexafluoraceton, PMVE (Perfluormethylvinylether), PEVE (Perfluorethylvinylether) und Mischungen davon.

4. Verwendung eines Schleppmittels zum Trennen von HF von einer Mischung umfassend HFC-1243zf, HF und mindestens eines von HFC-254fb oderHFC-254eb.

## Revendications

1. Procédé de séparation du HF d'un mélange comprenant du HFC-1243zf, du HF, et au moins l'un du HFC-254fb ou du HFC-254eb, ledit procédé comprenant:
a. l'addition d'un agent d'entraînement au mélange comprenant du HFC-1243zf, du HF, et au moins l'un du HFC-254fb ou du HFC-254eb formant ainsi un second mélange;
b. la distillation dudit second mélange dans une première étape de distillation pour former une première composition de distillat comprenant du HF et l'agent d'entraînement et une première composition de fonds comprenant du HFC-1243zf et au moins l'un du HFC-254fb ou du HFC-254eb;
c. la condensation de ladite première composition de distillat pour former deux phases liquides, étant (i) une phase riche en agent d'entraînement et (ii) une phase riche en HF; et
d. le recyclage de la phase riche en agent d'entraînement vers la première étape de distillation.

2. Procédé selon la revendication 1 comprenant en outre l'alimentation de la phase riche en HF vers une seconde étape de distillation et la formation d'une seconde composition de distillat comprenant un azéotrope d'agent d'entraînement et du HF et une seconde composition de fonds comprenant du HF essentiellement exempte d'agent d'entraînement.

3. Procédé selon la revendication 1, ledit agent d'entraînement étant sélectionné dans le groupe constitué:
a. des agents d'entraînement hydrocarbures comprenant au moins un composé sélectionné dans le groupe constitué: du méthane, de l'éthane, de l'éthylène, de l'acétylène, du vinylacétylène, du propane, du propylène, du propyne, du cyclopropane, du cyclopropène, du propadiène, du *n*-butane, de l'isobutane, du 1-butène, de l'isobutène, du 1,3-butadiène, du 2,2-diméthylpropane, du *cis*-2-butène, du *trans*-2-butène, du 1-butyne, du *n*-pentane, de l'isopentane, du néopentane, du cyclopentane, du 1-pentène, du 2-pentène, et de leurs mélanges;
b. des agents d'entraînement chlorocarbures sélectionnés dans le groupe constitué du chlorure de méthylène, du chlorure de méthyle, et de leurs mélanges;
c. des agents d'entraînement chlorurofluorurocarbones (CFC) comprenant au moins un composé sélectionné dans le groupe constitué: du dichlorodifluorométhane (CFC-12), 2-chloro-1,1,2-trifluoroéthylène, chloropentafluoroéthane (CFC-115), 1,2-dichloro-1,1,2,2-tétrafluoroéthane (CFC-114), 1,1-dichloro-1,2,2,2-tétrafluoroéthane (CFC-114a), 1,1,2-trichloro-1,2,2-trifluoroéthane (CFC-113), 1,1,1-trichloro-2,2,2-trifluoroéthane (CFC-113a), 1,1,2-trichloro-1,2,3,3,3-pentafluoropropane (CFC-215bb), 2,2-dichloro-1,1,1,3,3,3-hexafluoropropane (CFC-216aa), 1,2-dichloro-1,1,2,3,3,3-hexafluoropropane (CFC-216ba), 2-chloro-1,1,1,2,3,3,3-heptafluoropropane (CFC-217ba), 2-chloro-1,1,3,3,3-pentafluoropropène (CFC-1215xc), et de leurs mélanges;
d. des agents d'entraînement hydrurochlorurofluorurocarbones (HCFC) comprenant au moins un composé sélectionné dans le groupe constitué: du dichlorofluorométhane (HCFC-21), 1,1-dichloro-3,3,3-trifluoroéthane (HCFC-123), 1,1-dichloro-1-fluoroéthane (HCFC-141b), 2-chloro-1,1,1,2-tétrafluoroéthane (HCFC-124), 1-chloro-1,1,2,2-tétrafluoroéthane (HCFC-124a), 2-chloro-1,1,1-trifluoroéthane (HCFC-133a), 1-chloro-1,1-difluoroéthane (HCFC-142b), 2-chloro-1,1-difluoroéthylène (HCFC-1122), et de leurs mélanges;
e. des agents d'entraînement hydrurofluorurocarbones (HFC) comprenant au moins un composé sélectionné dans le groupe constitué: du 1,1,2-trifluoroéthylène (HFC-1123), 1,1-difluoroéthylène (HFC-1132a), 2,3,3,3-tétrafluoropropène (HFC-1234yf), et de leurs mélanges;
f. des agents d'entraînement perfluorocarbones (PFC) comprenant au moins un composé sélectionné dans le groupe constitué: de l'hexafluoroéthane (PFC-116), de l'octafluoropropane (PFC-218), du 1,1,1,4,4,4-hexafluoro-2-butyne (PFBY-2), de l'hexafluoropropylène (HFP, PFC-1216), de l'hexafluorocyclopropane (PFC-C216), de l'octafluorocyclobutane (PFC-C318), du décafluorobutane (PFC-31-10, tous les isomères), du 2,3-dichloro-1,1,1,4,4,4-hexafluoro-2-butène (PFC-1316mxx), de l'octafluoro-2-butène (PFC-1318my, *cis* et *trans),* de l'hexafluorobutadiène (PFC-2316), et de leurs mélanges;
g. des agents d'entraînement éther fluorés comprenant au moins un composé sélectionné dans le groupe constitué: de l'éther de trifluorométhyl-difluorométhyle (CF₃OCHF₂, HFOC-125E), de l'éther de 1,1-difluorodiméthyle, du tétrafluorodiméthyléther (HFOC-134E), du méthyl éther de difluorométhyle (CHF₂OCH₃, HFOC-152aE), du méthyl éther de pentafluoroéthyle, et de leurs mélanges; et
h. au moins un composé sélectionné dans le groupe constitué du HFPO (oxyde d'hexafluoropropylène), du SF₆, du chlore, de l'hexafluoroacétone, du PMVE (éther de perfluorométhylvinyle), du PEVE (éther de perfluoroéthylvinyle), et de leurs mélanges.

4. Utilisation d'un agent d'entraînement destiné à séparer le HF d'un mélange comprenant du HFC-1243zf, du HF, et au moins l'un du HFC-254fb ou du HFC-254eb.
